# EUROPEAN PATENT APPLICATION

(11) **EP 3 346 007 A1**
(43) Date of publication of application: **11.07.2018**
(21) Application number: 17150683.5
(22) Date of filing: 09.01.2017
(51) Int. Cl.: C12N 15/113, A61K 31/712, A61K 31/7125, C07H 21/00

(54) **IMMUNOSUPPRESSION-REVERTING OLIGONUCLEOTIDES INHIBITING THE EXPRESSION OF TDO**

(71) Applicant: Secarna Pharmaceuticals GmbH & Co. KG, 35037 Marburg (DE)
(72) Inventor: Klar, Richard, 35037 Marburg (DE); Jaschinski, Frank, 35037 Marburg (DE)
(74) Representative: V.O.

(57) **Abstract**

The present invention refers to an immunosuppression-reverting oligonucleotide hybridizing with a nucleic acid sequence of tryptophan-2,3-dioxygenase (TDO) and a pharmaceutical composition comprising such immunosuppression-reverting oligonucleotide and a pharmaceutically acceptable carrier, excipient and/or diluent. The oligonucleotide and the pharmaceutical composition, respectively, is used in a method for preventing and/or treating an autoimmune disorder, an immune disorder, a psychiatric disorder and/or cancer.

SEQUENCE LISTING

<110>  Secarna Pharmaceuticals GmbH & Co KG

<120>  Immunosuppression-reverting oligonucleotides inhibiting the
       expression of TDO

<130>  P113899EP00

<140>  EP 17150683.5
<141>  2017-01-09

<160>  90

<170>  PatentIn version 3.5

<210>  1
<211>  1703
<212>  DNA
<213>  Homo sapiens

<400>  1
   ggaaggtcaa tgatagcatc tgcctagagt caaacctccg tgcttctcag acagtgcctt      60
   ttcaccatga gtgggtgccc atttttagga aacaactttg gatatacttt taaaaaactc     120
   cccgtagaag gcagcgaaga agacaaatca caaactggtg tgaatagagc cagcaaagga     180
   ggtcttatct atgggaacta cctgcatttg gaaaaagttt tgaatgcaca agaactgcaa     240
   agtgaaacaa aaggaaataa aatccatgat gaacatcttt ttatcataac tcatcaagct     300
   tatgaactct ggtttaagca aatcctctgg gagttggatt ctgttcgaga gatctttcag     360
   aatggccatg tcagagatga aaggaacatg cttaaggttg tttctcggat gcaccgagtg     420
   tcagtgatcc tgaaactgct ggtgcagcag ttttccattc tggagacgat gacagccttg     480
   gacttcaatg acttcagaga gtacttatct ccagcatcag gcttccagag tttgcaattc     540
   cgactattag aaaacaagat aggtgttctt cagaacatga gagtccctta taacagaaga     600
   cattatcgtg ataacttcaa aggagaagaa aatgaactgc tacttaaatc tgagcaggaa     660
   aagacacttc tggaattagt ggaggcatgg ctggaaagaa ctccaggttt agagccacat     720
   ggatttaact tctggggaaa gcttgaaaaa aatatcacca gaggcctgga agaggaattc     780
   ataaggattc aggctaaaga agagtctgaa gaaaaagagg aacaggtggc tgaatttcag     840
   aagcaaaaag aggtgctact gtccttattt gatgagaaac gtcatgaaca tctccttagt     900
   aaaggtgaaa gacggctgtc atacagagca cttcagggag cattgatgat atatttttac     960
   agggaagagc ctaggttcca ggtgcctttt cagttgctga cttctcttat ggacatagat    1020
   tcactgatga ccaaatggag atataaccat gtgtgcatgg tgcacagaat gctgggcagc    1080
   aaagctggca ccggtggttc ctcaggctat cactacctgc gatcaactgt gagtgatagg    1140
   tacaaggtat ttgtagattt atttaatctt tcaacatacc tgattccccg acactggata    1200
   ccgaagatga acccaaccat tcacaaattt ctatatacag cagaatactg tgatagctcc    1260
   tacttcagca gtgatgaatc agattaaaat cgtctgcaaa atctatgaag aatactggtt 1320
   tcacagccta ttttttattt tctatggatt ttcataaata cagtttgaat atatgtatgc 1380
   atatattgtt cagcaccacg atgctctgat ttaattctag aaacaatttg attacctctt 1440
   gtttgtgaca agactaagca ttaagatgag aaagaataca tttaaatagt aacattgtac 1500
   atagggtgtt ttcctattaa aaattcagtt tcccctgaga cttaatgtaa ccacttaatg 1560
   taatcactat ctcattgttt catctttata aacttgtaaa cttcatctat ttcaaatatt 1620
   ttatgcagta cattatatta ttctgtacaa aggctttcaa acaaaatttt taaaataata 1680
   aagtattaat ctttctccct gta 1703

<210> 2
<211> 1646
<212> DNA
<213> Mus musculus

<400> 2
agcaaacctg tgtggtcctg agacacttca gtactatgag tgggtgcccg tttgcaggaa 60

acagtgtagg atacactttg aaaaacgtat ctatggagga caatgaagaa gacagagctc 120

aaactggtgt aaacagagcc agcaaaggag gacttatcta tggcaattac ttgcagttgg 180

aaaagatttt gaatgcgcaa gaacttcaga gtgaagtaaa agggaacaaa atccatgacg 240

agcacctatt catcataact catcaagctt atgaactttg gtttaaacaa atcctctggg 300

aactagattc tgttcgtgaa atcttccaaa atggccatgt cagggatgag aggaacatgc 360

tcaaggtgat agctcggatg catcgtgtgg tggtcatctt caagctcctg gtacagcagt 420

tctcggttct ggaaacaatg actgctttgg acttcaatga cttcagagaa tacctgtctc 480

cagcatcagg cttccagagt ctacagttcc ggctgctgga aaataagatt ggtgttcttc 540

agagcttgag agtcccttac aacaggaaac actatcgtga taactttgga ggagactaca 600

atgagctgtt gctgaaatca gagcaggagc agacgctgtt gcagctggtg gaggcatggc 660

tggaaagaac acctggttta gagccaaatg gatttaattt ctggggaaag tttgaaaaaa 720

atatcttgaa aggcctggaa gaagaatttc taaggattca ggctaaaacc gactctgaag 780

aaaaagagga acagatggca gagttccgga agcagaagga ggtgctgctc tgcttgtttg 840

atgagaagcg tcatgactac cttctgagta aaggtgaacg acgactgtca taccgtgcac 900

tccagggagc actgatgata tatttttaca gggaggagcc tcgattccag gtccctttcc 960

agctgctgac ctcacttatg gacattgaca cgctcatgac caaatggaga tataatcatg 1020

tgtgcatggt gcacagaatg ctgggcacca aggctggcac tgggggatcc tcaggctatc 1080

attacctgcg ttcaactgtg agcgacaggt acaaggtttt tgtggattta tttaacctct 1140

caacatatct ggttcccaga cactgggtac caaagatgaa tccgatcatt cacaaattcc 1200

tttacacagc cgagtacagt gacagctctt acttcagcag cgatgaatcg gattgagttc 1260

ttctgaacat cagtgaaaac tacaggattc tcagtcggct ttttataaat ttttatgaat 1320

acatgattgg tgtaatctat ttatatgtgt agttcagtgt tatgatgttt tggtccaatc 1380

ctggaaaaaa gtttatgatc ttgcatatca tgatggtgag cgattaggag gattaagcat 1440

tatgataact gatatagtaa aatgttagca tcatcgtaca tatgataaat tcttgctaca 1500

actcaattta ccctgacatt tacctctgta gaaccatttc atataattat taccttattg 1560

cttcatactt tataaagctt gttgagcagt tactttgtat tatagataca ataaatacta 1620

cccttctgta caaaaaaaaa aaaaaa 1646

<210> 3
<211> 14
<212> DNA
<213> Artificial Sequence

<220>
<223> antisense oligonucleotide hybridizing with human TDO2

<400> 3
tgtatgacag ccgt 14

<210> 4
<211> 17
<212> DNA
<213> Artificial Sequence

<220>
<223> antisense oligonucleotide hybridizing with human TDO2

<400> 4
atcgtggtgc tgaacaa 17

<210> 5
<211> 13
<212> DNA
<213> Artificial Sequence

<220>
<223> antisense oligonucleotide hybridizing with human TDO2

<400> 5
gtatccagtg tcg 13

<210> 6
<211> 14
<212> DNA
<213> Artificial Sequence

<220>
<223> antisense oligonucleotide hybridizing with human TDO2

<400> 6

ctcgaacaga atcc                                                          14

<210>  7
<211>  14
<212>  DNA
<213>  Artificial Sequence

<220>
<223>  antisense oligonucleotide hybridizing with human TDO2

<400>  7
tctcgaacag aatc                                                          14

<210>  8
<211>  14
<212>  DNA
<213>  Artificial Sequence

<220>
<223>  antisense oligonucleotide hybridizing with human TDO2

<400>  8
tcggaattgc aaac                                                          14

<210>  9
<211>  14
<212>  DNA
<213>  Artificial Sequence

<220>
<223>  antisense oligonucleotide hybridizing with human TDO2

<400>  9
gaagttatca cgat                                                          14

<210>  10
<211>  14
<212>  DNA
<213>  Artificial Sequence

<220>
<223>  antisense oligonucleotide hybridizing with human TDO2

<400>  10
accggtgcca gctt                                                          14

<210>  11
<211>  14
<212>  DNA
<213>  Artificial Sequence

<220>
<223>  antisense oligonucleotide hybridizing with human TDO2

<400>  11
catcttcggt atcc                                                          14

<210>  12
<211>  14
<212>  DNA
<213>  Artificial Sequence

<220>
<223>  antisense oligonucleotide hybridizing with human TDO2

<400>  12
tcgtggtgct gaac                                                          14


<210>  13
<211>  15
<212>  DNA
<213>  Artificial Sequence

<220>
<223>  antisense oligonucleotide hybridizing with human TDO2

<400>  13
tatcattgac cttcc                                                         15


<210>  14
<211>  15
<212>  DNA
<213>  Artificial Sequence

<220>
<223>  antisense oligonucleotide hybridizing with human TDO2

<400>  14
acccactcat ggtga                                                         15


<210>  15
<211>  15
<212>  DNA
<213>  Artificial Sequence

<220>
<223>  antisense oligonucleotide hybridizing with human TDO2

<400>  15
gataagacct ccttt                                                         15


<210>  16
<211>  15
<212>  DNA
<213>  Artificial Sequence

<220>
<223>  antisense oligonucleotide hybridizing with human TDO2

<400>  16
ccaaatgcag gtagt                                                         15


<210>  17
<211>  15
<212>  DNA

<213>  Artificial Sequence

<220>
<223>  antisense oligonucleotide hybridizing with human TDO2

<400>  17
ggatttgctt aaacc                                                        15


<210>  18
<211>  15
<212>  DNA
<213>  Artificial Sequence

<220>
<223>  antisense oligonucleotide hybridizing with human TDO2

<400>  18
ctctcgaaca gaatc                                                        15


<210>  19
<211>  15
<212>  DNA
<213>  Artificial Sequence

<220>
<223>  antisense oligonucleotide hybridizing with human TDO2

<400>  19
tccgagaaac aacct                                                        15


<210>  20
<211>  15
<212>  DNA
<213>  Artificial Sequence

<220>
<223>  antisense oligonucleotide hybridizing with human TDO2

<400>  20
gatcactgac actcg                                                        15


<210>  21
<211>  15
<212>  DNA
<213>  Artificial Sequence

<220>
<223>  antisense oligonucleotide hybridizing with human TDO2

<400>  21
tcggaattgc aaact                                                        15


<210>  22
<211>  15
<212>  DNA
<213>  Artificial Sequence

<220>

<223>  antisense oligonucleotide hybridizing with human TDO2

<400>  22
acgataatgt cttct                                                        15


<210>  23
<211>  15
<212>  DNA
<213>  Artificial Sequence

<220>
<223>  antisense oligonucleotide hybridizing with human TDO2

<400>  23
atgtggctct aaacc                                                        15


<210>  24
<211>  15
<212>  DNA
<213>  Artificial Sequence

<220>
<223>  antisense oligonucleotide hybridizing with human TDO2

<400>  24
tagcctgaat cctta                                                        15


<210>  25
<211>  15
<212>  DNA
<213>  Artificial Sequence

<220>
<223>  antisense oligonucleotide hybridizing with human TDO2

<400>  25
gacagtagca cctct                                                        15


<210>  26
<211>  15
<212>  DNA
<213>  Artificial Sequence

<220>
<223>  antisense oligonucleotide hybridizing with human TDO2

<400>  26
gatgttcatg acgtt                                                        15


<210>  27
<211>  15
<212>  DNA
<213>  Artificial Sequence

<220>
<223>  antisense oligonucleotide hybridizing with human TDO2

<400>  27

tctgtatgac agccg                                                        15

<210>  28
<211>  15
<212>  DNA
<213>  Artificial Sequence

<220>
<223>  antisense oligonucleotide hybridizing with human TDO2

<400>  28
agtgaatcta tgtcc                                                        15

<210>  29
<211>  15
<212>  DNA
<213>  Artificial Sequence

<220>
<223>  antisense oligonucleotide hybridizing with human TDO2

<400>  29
catggttata tctcc                                                        15

<210>  30
<211>  15
<212>  DNA
<213>  Artificial Sequence

<220>
<223>  antisense oligonucleotide hybridizing with human TDO2

<400>  30
accggtgcca gcttt                                                        15

<210>  31
<211>  15
<212>  DNA
<213>  Artificial Sequence

<220>
<223>  antisense oligonucleotide hybridizing with human TDO2

<400>  31
gaaccaccgg tgcca                                                        15

<210>  32
<211>  15
<212>  DNA
<213>  Artificial Sequence

<220>
<223>  antisense oligonucleotide hybridizing with human TDO2

<400>  32
tcggtatcca gtgtc                                                        15

<210>  33
<211>  15
<212>  DNA
<213>  Artificial Sequence

<220>
<223>  antisense oligonucleotide hybridizing with human TDO2

<400>  33
aggagctatc acagt                                                        15


<210>  34
<211>  15
<212>  DNA
<213>  Artificial Sequence

<220>
<223>  antisense oligonucleotide hybridizing with human TDO2

<400>  34
tcagagcatc gtggt                                                        15


<210>  35
<211>  15
<212>  DNA
<213>  Artificial Sequence

<220>
<223>  antisense oligonucleotide hybridizing with human TDO2

<400>  35
ccctatgtac aatgt                                                        15


<210>  36
<211>  16
<212>  DNA
<213>  Artificial Sequence

<220>
<223>  antisense oligonucleotide hybridizing with human TDO2

<400>  36
tctctcgaac agaatc                                                       16


<210>  37
<211>  16
<212>  DNA
<213>  Artificial Sequence

<220>
<223>  antisense oligonucleotide hybridizing with human TDO2

<400>  37
atccgagaaa caacct                                                       16


<210>  38
<211>  16
<212>  DNA

<213>  Artificial Sequence

<220>
<223>  antisense oligonucleotide hybridizing with human TDO2

<400>  38
gacactcggt gcatcc                                                       16


<210>  39
<211>  16
<212>  DNA
<213>  Artificial Sequence

<220>
<223>  antisense oligonucleotide hybridizing with human TDO2

<400>  39
atcactgaca ctcggt                                                       16


<210>  40
<211>  16
<212>  DNA
<213>  Artificial Sequence

<220>
<223>  antisense oligonucleotide hybridizing with human TDO2

<400>  40
gtcggaattg caaact                                                       16


<210>  41
<211>  16
<212>  DNA
<213>  Artificial Sequence

<220>
<223>  antisense oligonucleotide hybridizing with human TDO2

<400>  41
gagatgttca tgacgt                                                       16


<210>  42
<211>  16
<212>  DNA
<213>  Artificial Sequence

<220>
<223>  antisense oligonucleotide hybridizing with human TDO2

<400>  42
gtatgacagc cgtctt                                                       16


<210>  43
<211>  16
<212>  DNA
<213>  Artificial Sequence

<220>

<223>  antisense oligonucleotide hybridizing with human TDO2

<400>  43
ctctgtatga cagccg                                                        16


<210>  44
<211>  16
<212>  DNA
<213>  Artificial Sequence

<220>
<223>  antisense oligonucleotide hybridizing with human TDO2

<400>  44
accggtgcca gctttg                                                        16


<210>  45
<211>  16
<212>  DNA
<213>  Artificial Sequence

<220>
<223>  antisense oligonucleotide hybridizing with human TDO2

<400>  45
gatagcctga ggaacc                                                        16


<210>  46
<211>  16
<212>  DNA
<213>  Artificial Sequence

<220>
<223>  antisense oligonucleotide hybridizing with human TDO2

<400>  46
ttcggtatcc agtgtc                                                        16


<210>  47
<211>  16
<212>  DNA
<213>  Artificial Sequence

<220>
<223>  antisense oligonucleotide hybridizing with human TDO2

<400>  47
ttcatcttcg gtatcc                                                        16


<210>  48
<211>  17
<212>  DNA
<213>  Artificial Sequence

<220>
<223>  antisense oligonucleotide hybridizing with human TDO2

<400>  48

gctatcattg accttcc                                                    17

<210>  49
<211>  17
<212>  DNA
<213>  Artificial Sequence

<220>
<223>  antisense oligonucleotide hybridizing with human TDO2

<400>  49
ctctcgaaca gaatcca                                                    17

<210>  50
<211>  17
<212>  DNA
<213>  Artificial Sequence

<220>
<223>  antisense oligonucleotide hybridizing with human TDO2

<400>  50
tccgagaaac aacctta                                                    17

<210>  51
<211>  17
<212>  DNA
<213>  Artificial Sequence

<220>
<223>  antisense oligonucleotide hybridizing with human TDO2

<400>  51
actgacactc ggtgcat                                                    17

<210>  52
<211>  17
<212>  DNA
<213>  Artificial Sequence

<220>
<223>  antisense oligonucleotide hybridizing with human TDO2

<400>  52
gtcggaattg caaactc                                                    17

<210>  53
<211>  17
<212>  DNA
<213>  Artificial Sequence

<220>
<223>  antisense oligonucleotide hybridizing with human TDO2

<400>  53
gttatcacga taatgtc                                                    17

<210>  54
<211>  17
<212>  DNA
<213>  Artificial Sequence

<220>
<223>  antisense oligonucleotide hybridizing with human TDO2

<400>  54
ataaggacag tagcacc                                                      17


<210>  55
<211>  17
<212>  DNA
<213>  Artificial Sequence

<220>
<223>  antisense oligonucleotide hybridizing with human TDO2

<400>  55
gttcatgacg tttctca                                                      17


<210>  56
<211>  17
<212>  DNA
<213>  Artificial Sequence

<220>
<223>  antisense oligonucleotide hybridizing with human TDO2

<400>  56
tgtatgacag ccgtctt                                                      17


<210>  57
<211>  17
<212>  DNA
<213>  Artificial Sequence

<220>
<223>  antisense oligonucleotide hybridizing with human TDO2

<400>  57
tgtccataag agaagtc                                                      17


<210>  58
<211>  17
<212>  DNA
<213>  Artificial Sequence

<220>
<223>  antisense oligonucleotide hybridizing with human TDO2

<400>  58
accggtgcca gctttgc                                                      17


<210>  59
<211>  17
<212>  DNA

<213>  Artificial Sequence

<220>
<223>  antisense oligonucleotide hybridizing with human TDO2

<400>  59
acctatcact cacagtt                                                    17


<210>  60
<211>  17
<212>  DNA
<213>  Artificial Sequence

<220>
<223>  antisense oligonucleotide hybridizing with human TDO2

<400>  60
ttcatcttcg gtatcca                                                    17


<210>  61
<211>  17
<212>  DNA
<213>  Artificial Sequence

<220>
<223>  antisense oligonucleotide hybridizing with human TDO2

<400>  61
ccctatgtac aatgtta                                                    17


<210>  62
<211>  18
<212>  DNA
<213>  Artificial Sequence

<220>
<223>  control antisense oligonucleotide

<400>  62
cgtttaggct atgtactt                                                   18


<210>  63
<211>  15
<212>  DNA
<213>  Artificial Sequence

<220>
<223>  antisense oligonucleotide hybridizing with murine and/or rat TDO2

<400>  63
tcgtcgttca ccttt                                                      15


<210>  64
<211>  16
<212>  DNA
<213>  Artificial Sequence

<220>

<223>  antisense oligonucleotide hybridizing with murine and/or rat TDO2

<400>  64
atcacgatag tgtttc                                                    16


<210>  65
<211>  16
<212>  DNA
<213>  Artificial Sequence

<220>
<223>  antisense oligonucleotide hybridizing with murine and/or rat TDO2

<400>  65
gttatcacga tagtgt                                                    16


<210>  66
<211>  16
<212>  DNA
<213>  Artificial Sequence

<220>
<223>  antisense oligonucleotide hybridizing with murine and/or rat TDO2

<400>  66
tcgtcgttca ccttta                                                    16


<210>  67
<211>  17
<212>  DNA
<213>  Artificial Sequence

<220>
<223>  antisense oligonucleotide hybridizing with murine and/or rat TDO2

<400>  67
atcacgatag tgtttcc                                                   17


<210>  68
<211>  15
<212>  DNA
<213>  Artificial Sequence

<220>
<223>  antisense oligonucleotide hybridizing with murine and/or rat TDO2

<400>  68
gtagtcatga cgctt                                                     15


<210>  69
<211>  15
<212>  DNA
<213>  Artificial Sequence

<220>
<223>  antisense oligonucleotide hybridizing with murine and/or rat TDO2

<400>  69

cagtcgtcgt tcacc                                                    15


<210>  70
<211>  15
<212>  DNA
<213>  Artificial Sequence

<220>
<223>  antisense oligonucleotide hybridizing with murine and/or rat TDO2

<400>  70
acagtcgtcg ttcac                                                    15


<210>  71
<211>  16
<212>  DNA
<213>  Artificial Sequence

<220>
<223>  antisense oligonucleotide hybridizing with murine and/or rat TDO2

<400>  71
ccatagataa gtcctc                                                   16


<210>  72
<211>  16
<212>  DNA
<213>  Artificial Sequence

<220>
<223>  antisense oligonucleotide hybridizing with murine and/or rat TDO2

<400>  72
cgagaactgc tgtacc                                                   16


<210>  73
<211>  16
<212>  DNA
<213>  Artificial Sequence

<220>
<223>  antisense oligonucleotide hybridizing with murine and/or rat TDO2

<400>  73
cgtcgttcac ctttac                                                   16


<210>  74
<211>  16
<212>  DNA
<213>  Artificial Sequence

<220>
<223>  antisense oligonucleotide hybridizing with murine and/or rat TDO2

<400>  74
agtcgtcgtt cacctt                                                   16

<210>  75
<211>  16
<212>  DNA
<213>  Artificial Sequence

<220>
<223>  antisense oligonucleotide hybridizing with murine and/or rat TDO2

<400>  75
cagtcgtcgt tcacct                                                    16


<210>  76
<211>  16
<212>  DNA
<213>  Artificial Sequence

<220>
<223>  antisense oligonucleotide hybridizing with murine and/or rat TDO2

<400>  76
ggtatgacag tcgtcg                                                    16


<210>  77
<211>  16
<212>  DNA
<213>  Artificial Sequence

<220>
<223>  antisense oligonucleotide hybridizing with murine and/or rat TDO2

<400>  77
cacggtatga cagtcg                                                    16


<210>  78
<211>  16
<212>  DNA
<213>  Artificial Sequence

<220>
<223>  antisense oligonucleotide hybridizing with murine and/or rat TDO2

<400>  78
tgcacggtat gacagt                                                    16


<210>  79
<211>  16
<212>  DNA
<213>  Artificial Sequence

<220>
<223>  antisense oligonucleotide hybridizing with murine and/or rat TDO2

<400>  79
ggagtgcacg gtatga                                                    16


<210>  80
<211>  16
<212>  DNA

<213>  Artificial Sequence

<220>
<223>  antisense oligonucleotide hybridizing with murine and/or rat TDO2

<400>  80
gtacctgtcg ctcaca                                                       16


<210>  81
<211>  16
<212>  DNA
<213>  Artificial Sequence

<220>
<223>  antisense oligonucleotide hybridizing with murine and/or rat TDO2

<400>  81
cttgtacctg tcgctc                                                       16


<210>  82
<211>  16
<212>  DNA
<213>  Artificial Sequence

<220>
<223>  antisense oligonucleotide hybridizing with murine and/or rat TDO2

<400>  82
accttgtacc tgtcgc                                                       16


<210>  83
<211>  16
<212>  DNA
<213>  Artificial Sequence

<220>
<223>  antisense oligonucleotide hybridizing with murine and/or rat TDO2

<400>  83
aatgatcgga ttcatc                                                       16


<210>  84
<211>  16
<212>  DNA
<213>  Artificial Sequence

<220>
<223>  antisense oligonucleotide hybridizing with murine and/or rat TDO2

<400>  84
gattcatcgc tgctga                                                       16


<210>  85
<211>  17
<212>  DNA
<213>  Artificial Sequence

<220>

<223>  antisense oligonucleotide hybridizing with murine and/or rat TDO2

<400>  85
catagataag tcctcct                                                    17


<210>  86
<211>  17
<212>  DNA
<213>  Artificial Sequence

<220>
<223>  antisense oligonucleotide hybridizing with murine and/or rat TDO2

<400>  86
tcgtcgttca cctttac                                                    17


<210>  87
<211>  17
<212>  DNA
<213>  Artificial Sequence

<220>
<223>  antisense oligonucleotide hybridizing with murine and/or rat TDO2

<400>  87
cagtcgtcgt tcacctt                                                    17


<210>  88
<211>  17
<212>  DNA
<213>  Artificial Sequence

<220>
<223>  antisense oligonucleotide hybridizing with murine and/or rat TDO2

<400>  88
acagtcgtcg ttcacct                                                    17


<210>  89
<211>  17
<212>  DNA
<213>  Artificial Sequence

<220>
<223>  antisense oligonucleotide hybridizing with murine and/or rat TDO2

<400>  89
tgtgaatgat cggattc                                                    17


<210>  90
<211>  18
<212>  DNA
<213>  Artificial Sequence

<220>
<223>  antisense oligonulcoetide negative control

<400>  90

cgtttaggct atgtactt 18

## Description

The present disclosure refers to an immunosuppression-reverting oligonucleotide hybridizing with a nucleic acid sequence of tryptophan-2,3-dioxygenase (TDO) such as TDO2 and a pharmaceutical composition comprising such immunosuppression-reverting oligonucleotide and a pharmaceutically acceptable carrier, excipient and/or diluent.

### Technical background

In recent years the treatment of several different diseases such as malignant tumors was very successful by application of immune therapy, in particular by inhibitors of so called "immune checkpoints". These checkpoints are molecules in the immune system that either turn up (co-stimulatory molecules) or down a signal. The concept of the therapeutic approach is based on the activation of endogenous anti-tumor immune reactions. Many cancers for example protect themselves from the immune system by inhibiting T cell and NK cell activity, respectively. Immune checkpoint modulators, i.e., stimulators or inhibitors are for example directed to one or more of CTLA-4, PD-1, PD-L1, LAG-3, VISTA, A2AR, BTLA, IDO (e.g. IDO1), CD39, CD73, STAT3, TIM-3, MICA, NKG2A, KIR, TIGIT, CD96, TGF-beta, Ox40, GITR, CD27, CD160, 2B4 and 4-1BB.

Tryptophan for example is an amino acid which is essential for cell proliferation and survival. It is required for the biosynthesis of the neurotransmitter serotonin, the synthesis of the cofactor nicotinamide adenine dinucleotide (NAD), and is an important factor in the suppression of the immune system response to tumors. Depletion of tryptophan and production of kynurenines is associated with adverse effects on the proliferation and function of lymphocytes and diminished immune system response.

The enzyme tryptophan-2,3-dioxygenase (TDO) and indoleamine-2,3-dioxygenase are cytosolic heme dioxygenases that catalyze the oxidative cleavage of the C2-C3 bond of the indolic ring of L-tryptophan (L-Trp) which is the first and rate-limiting step of the kynurenine pathway of tryptophan catabolism. Despite catalyzing the same biochemical reaction and sharing relatively conserved active site regions, TDO and IDO share an overall amino acid sequence identity of not more than 10 % (Dolusic E et al., J Medical Chemistry, 2011). TDO is homotetrameric and almost exclusively expressed in the liver, whereas IDO is monomeric and extrahepatic. In addition, Dolusic E et al. and Pilotte L et al., PNAS, 2012 reported that TDO is expressed in tumor cells such as melanoma, colorectal, bladder, hepatic, breast and lung cancers as well as in brain tumor cells (Opitz CA et al., Nature, 2011). TDO is highly specific for L-Trp and its derivatives substituted in the 5- and 6-positions of the indole ring. TDO is induced for example by tryptophan, glucocorticoides and kynurenine, whereas IDO is inducible for example by inflammatory stimuli such as interferon-gamma. The enzymatic activity of TDO and IDO, leads to the conversion of tryptophan to kynurenines and results in lack of tryptophan and severe immunosuppressive effects mediated by kynurenines. These effects result in suppression for example of tumor-reactive T-cells and natural killer (NK) cells for example with regard to cell proliferation, cytokine secretion and/or cytotoxic reactivity. Moreover, TDO has been implicated in neurologic and psychiatric disorders including mood disorders such as anxiety and depression.

Small molecules such as (E)-6-fluoro-3-[2-(3-pyridyl)vinyl]-1H-indole (680C91; Salter M et al., Biochem. Pharmacol., 1995) inhibiting TDO activity have been developed and tested in preclinical trials. However, the activity of the small molecules and their *in vivo* half-life is limited.

Immune therapies have resulted in long-term remission, but only of small patient groups so far. The reason may be that numerous immune checkpoints and optionally further immunosuppressive mechanisms are involved in the interaction between for example the immune system and the tumor cells. The combination of immune checkpoints and potential other mechanisms may vary depending on the tumor and individual conditions of a subject to escape the body's defenses.

For the inhibition of several immunosuppressive mechanisms common approaches using an antibody and/or a small molecule are not or hardly suitable as the molecular target is located intracellularly or does not have enzymatic activity. Accordingly, an agent which is safe and effective in inhibiting the function of an "immune checkpoint" such as TDO would be an important addition for the treatment of patients suffering from diseases or conditions affected for example by the activity of this enzyme.

Oligonucleotides of the present invention are very successful in the inhibition of the expression and activity of TDO, respectively. The mode of action of an oligonucleotide differs from the mode of action of an antibody or small molecule, and oligonucleotides are highly advantageous regarding for example
(i) the better penetration of tumor tissue in solid tumors compared to antibodies,
(ii) the blocking of multiple functions and activities, respectively, of a target,
(iii) the combination of oligonucleotides with each other or an antibody or a small molecule, and
(iv) the inhibition of intracellular effects which are not accessible for an antibody or targetable via a small molecule.
Oligonucleotides of the present invention are advantageous in comparison to first generation oligonucleotides due to their higher stability, stronger target affinity and potency and due to their independence from delivery reagents to achieve target suppression in cells.

### Summary

The present invention refers to an oligonucleotide such as an immunosuppression-reverting oligonucleotide comprising about 10 to 20 nucleotides, wherein at least one of the nucleotides is modified. The oligonucleotide hybridizes for example with a nucleic acid sequence of tryptophan-2,3-dioxygenase (TDO2) of SEQ ID NO.1 (human) and/or SEQ ID No.2 (mouse). The modified nucleotide is for example selected from the group consisting of a bridged nucleic acid (e.g., LNA, cET, ENA, 2'Fluoro modified nucleotide, 2'O-Methyl modified nucleotide or a combination thereof). In some embodiments, the oligonucleotide inhibits at least 50 % of the TDO2 expression and in some embodiments the oligonucleotide inhibits the expression of TDO2 at a nanomolar concentration.

The present invention is further directed to a pharmaceutical composition comprising an immunosuppression-reverting oligonucleotide of the present invention and optionally a pharmaceutically acceptable carrier, excipient, diluent or a combination thereof. In some embodiments, this pharmaceutical composition additionally comprises a chemotherapeutic such as platinum or gemcitabine, another oligonucleotide, an antibody and/or a small molecule which is for example effective in tumor treatment.

In some embodiments, the oligonucleotide of the present invention is in combination with another oligonucleotide, an antibody and/or a small molecule, either each of these compounds separate or combined in a pharmaceutical composition, wherein the oligonucleotide of the present invention inhibits the expression of an immune suppressive factor, and the antibody and/or the small molecule inhibits or stimulates an immune modulatory factor. The immune suppressive factor and/or the immune modulatory factor is for example CTLA-4, PD-1, PD-L1, LAG-3, VISTA, A2AR, BTLA, CD39, CD73, STAT3, IDO (e.g., IDO1), TIM-3, MICA, NKG2A, KIR, TIGIT, CD96, TGF-beta, Ox40, GITR, CD27, CD160, 2B4, 4-1BB, or a combination thereof.

Furthermore, the present invention relates to the use of the oligonucleotide or the pharmaceutical composition of the present invention in a method of preventing and/or treating a disorder, where TDO imbalance is involved. In some embodiments, the disorder is for example an autoimmune disorder, an immune disorder, a psychiatric disorder, an infectious liver disease, and/or cancer. In some embodiments, the oligonucleotide or the pharmaceutical composition of the present invention is for example administered locally or systemically.

All documents cited or referenced herein ("herein cited documents"), and all documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention. More specifically, all referenced documents are incorporated by reference to the same extent as if each individual document was specifically and individually indicated to be incorporated by reference.

### Description of figures

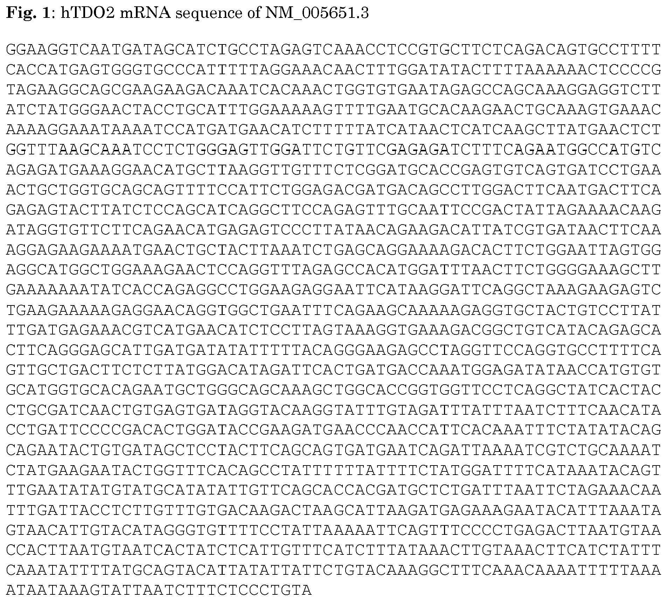

**Fig. 1** shows the mRNA sequence of human (h) TDO2 (SEQ ID No. 1); reference NM_005651.3.

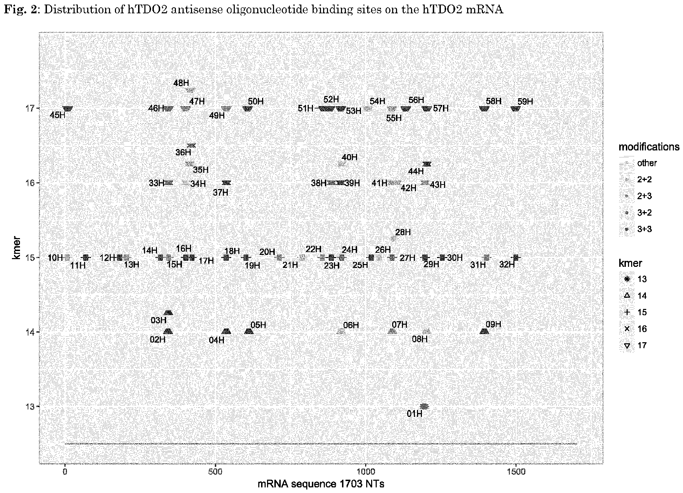

**Fig. 2** depicts the distribution of hTDO2 antisense oligonucleotide binding sites on the hTDO2 mRNA of SEQ ID No. 1 as well as their modification(s) and length. hTDO2 antisense oligonucleotides were aligned to the hTDO2 mRNA sequence. The different grayscales indicate the different LNA modifications and symbols indicate the different length of the antisense oligonucleotides.

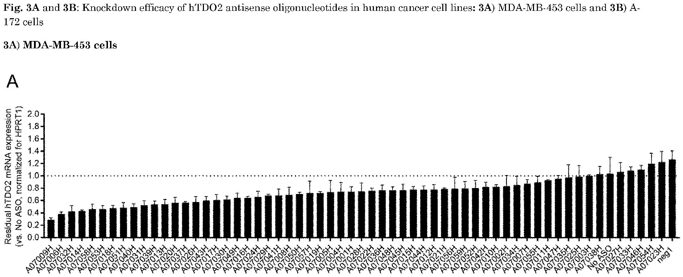

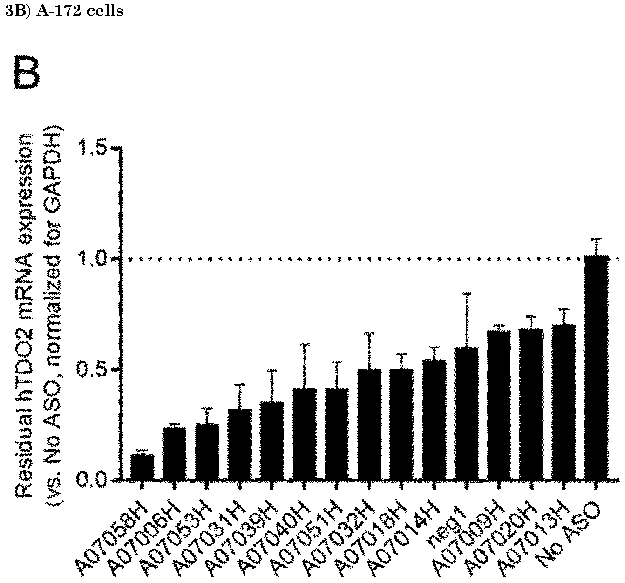

**Fig. 3A** and **3B** depict knockdown efficacy of hTDO2 antisense oligonucleotides in human cancer cell lines. In **Fig. 3A** human mammary carcinoma cells (MDA-MB-453 cells) were treated for 3 days with the indicated antisense oligonucleotides at a concentration of 5µM. In **Fig. 3B** human glioblastoma cells (A-172 cells) were treated for 4 days with selected antisense oligonucleotides at a concentration of 5µM, whereas antisense oligonucleotides were renewed at day 2. As negative control, cells were treated with neg1, an antisense oligonucleotide having the sequence CGTTTAGGCTATGTACTT (described in WO2014154843 A1). mRNA expression was analyzed at the end of the experiment by QuantiGene assay. Residual hTDO2 mRNA expression relative to untreated cells is depicted. Expression values were normalized to expression of the housekeeping gene HPRT1 (MDA-MB-453 cells) or GAPDH (A-172 cells), respectively.

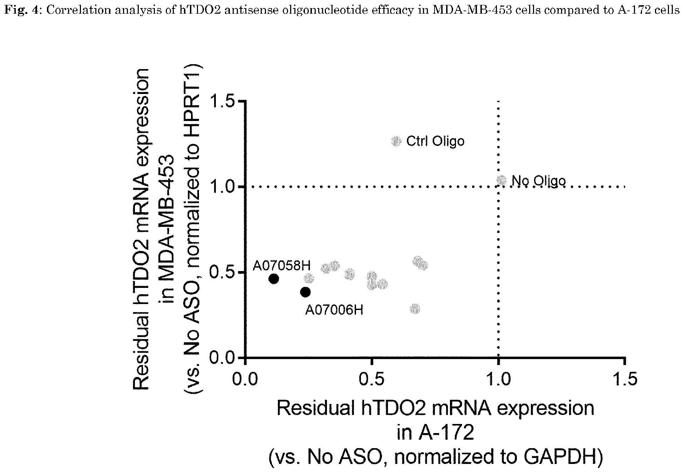

**Fig. 4** shows a correlation analysis of the efficacy of selected antisense oligonucleotides in MDA-MB-453 and A-172 cells.

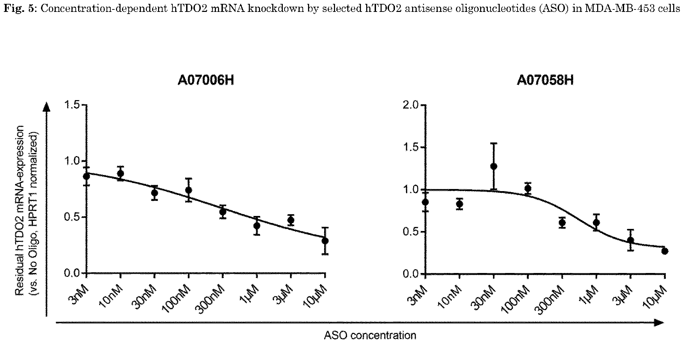

**Fig. 5** shows concentration-dependent hTDO2 mRNA knockdown by selected hTDO2 antisense oligonucleotides in MDA-MB-453 cells which were A07006H (SEQ ID No.3) and A07058H (SEQ ID No.4). MDA-MB-453 cells were treated for 3 days with the indicated concentration of the respective antisense oligonucleotide. Residual hTDO2 expression is depicted compared to untreated control cells. hTDO2 mRNA expression values were normalized to expression of the housekeeping gene HPRT1.
   Concentration-dependent target knockdown was used for calculation of IC₅₀ values shown in Table5.

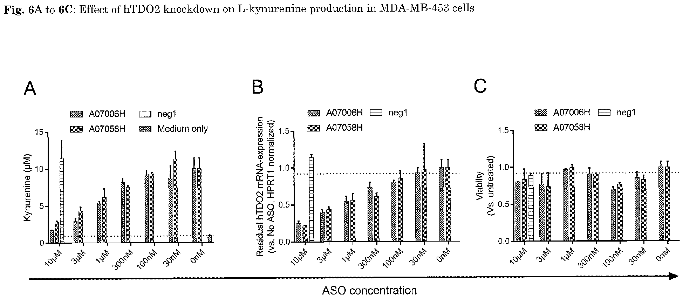

**Fig. 6A** to **6C** depict a concentration-dependent hTDO2 mRNA- and block of L-kynurenine production by A07006H (SEQ ID No.3) and A07058H (SEQ ID No.4) in MDA-MB-453 cells. MDA-MB-453 cells were treated for 3 days with the indicated concentrations of A07006H and A07058H, respectively, or control (neg1) antisense oligonucleotide. Medium was replaced and supplemented with L-Tryptophan, and Kynurenine was measured by ELISA in the supernatant after 24h (**Fig**. **6A**). Furthermore, hTDO2 knockdown efficacy on the mRNA level (**Fig. 6B**) and cell viability (**Fig. 6C**) was analyzed after 24h. Relative expression/viability compared to untreated control cells (=1) is depicted.

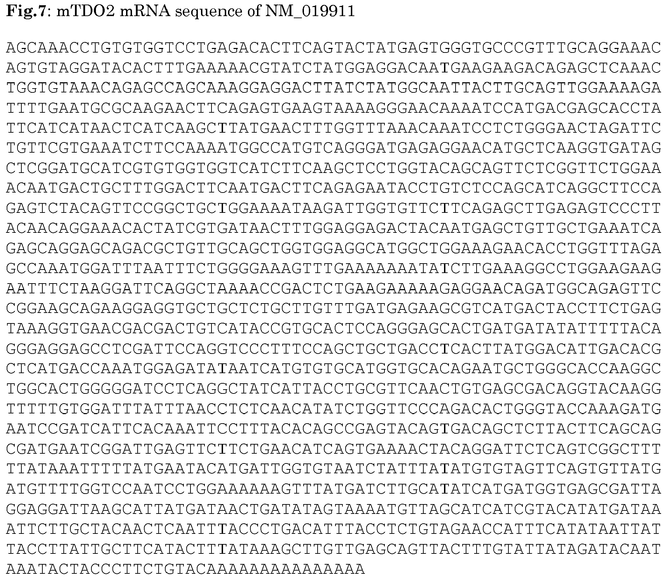

**Fig. 7** shows the mRNA sequence of murine (m) TDO2 (SEQ ID No. 2); reference NM_019911.

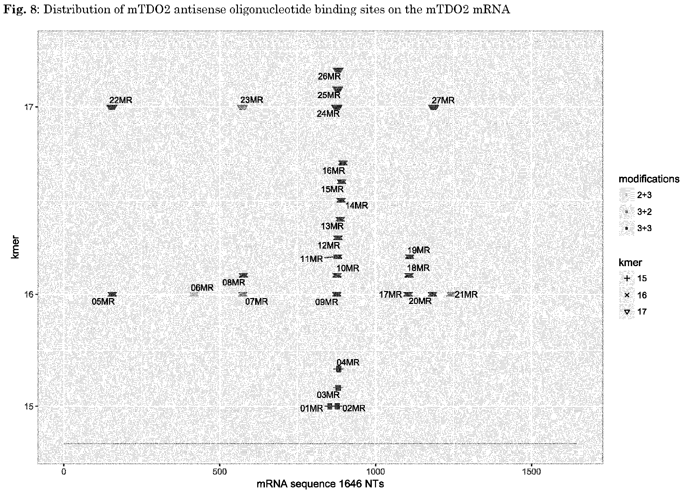

**Fig. 8** shows the distribution of mTDO2 antisense oligonucleotide binding sites on the mTDO2 mRNA of SEQ ID No. 2 (NM_019911) as well as their modification(s) and length. mTDO2 antisense oligonucleotide sequences were aligned to the mTDO2 mRNA sequence. The different grayscales indicate the different LNA modifications and symbols indicate the different length of the antisense oligonucleotides.

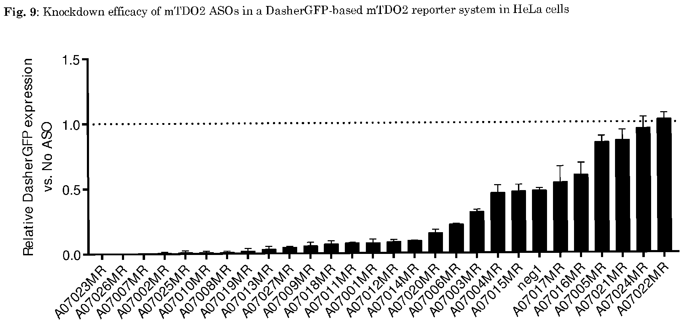

**Fig. 9** shows mTDO2 mRNA knockdown efficacy of mTDO2 antisense oligonucleotides in a DasherGFP reporter system in HeLa cells. HeLa cells were transfected with a reporter construct coding for the fluorescent protein DasherGFP and a fragment of the murine TDO2 gene and treated for 24 hours with 100 nM of the respective antisense oligonucleotide. As negative control, cells were treated with neg1, an antisense oligonucleotide having the sequence CGTTTAGGCTATGTACTT. Residual DasherGFP fluorescence relative to untreated cells is depicted.

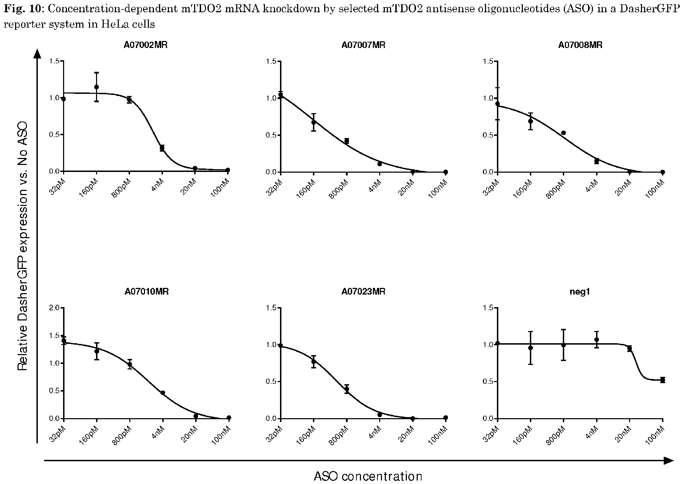

**Fig. 10** shows concentration-dependent mTDO2 mRNA knockdown by selected mTDO2 antisense oligonucleotides in a DasherGFP reporter system in HeLa cells. HeLa cells were transfected with a reporter construct coding for DasherGFP and a fragment of the murine TDO2 gene. Cells were treated with the respective antisense oligonucleotide at different concentrations for 24 hours. Residual DasherGFP fluorescence relative to untreated cells is depicted.

### Detailed description

The present invention provides for the first time human and murine oligonucleotides which hybridize with mRNA sequences of tryptophan-2,3-dioxygenase (TDO) such as TDO2 and inhibit the expression and activity, respectively, of TDO. In consequence, the level of tryptophan increases and the level of metabolites of tryptophan such as kynurenine decreases. Thus, the oligonucleotides of the present invention represent an interesting and highly efficient tool for use in a method of preventing and/or treating disorders, where the TDO such as the TDO2 expression and activity, respectively, is increased.

In the following, the elements of the present invention will be described in more detail. These elements are listed with specific embodiments, however, it should be understood that they may be combined in any manner and in any number to create additional embodiments. The variously described examples and embodiments should not be construed to limit the present invention to only the explicitly described embodiments. This description should be understood to support and encompass embodiments which combine the explicitly described embodiments with any number of the disclosed elements. Furthermore, any permutations and combinations of all described elements in this application should be considered disclosed by the description of the present application unless the context indicates otherwise.

Throughout this specification and the claims, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by the context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example"), provided herein is intended merely to better illustrate the invention and does not pose a limitation on the scope of the invention otherwise claimed. No language in the specification should be construed as indicating any non-claimed element essential to the practice of the invention.

Oligonucleotides of the present invention are for example antisense oligonucleotides consisting of or comprising 10 to 25 nucleotides, 10 to 15 nucleotides, 15 to 20 nucleotides, 12 to 18 nucleotides, or 14 to 17 nucleotides. The oligonucleotides for example consist of or comprise 10, 11, 12, 13, 14, 15, 16, 17, 18, 19, 20 or 25 nucleotides. The oligonucleotides of the present invention comprise at least one nucleotide which is modified. The modified nucleotide is for example a bridged nucleotide such as a locked nucleic acid (LNA, e.g., 2',4'-LNA), cET, ENA, a 2'Fluoro modified nucleotide, a 2'O-Methyl modified nucleotide or a combination thereof. In some embodiments, the oligonucleotide of the present invention comprises nucleotides having the same or different modifications. In some embodiments the oligonucleotide of the present invention further comprises a modified phosphate backbone, wherein the phosphate is for example a phosphorothioate.

The oligonucleotide of the present invention comprises the one or more modified nucleotide at the 3'- and/or 5'- end of the oligonucleotide and/or at any position within the oligonucleotide, wherein modified nucleotides follow in a row of 1, 2, 3, 4, 5, or 6 modified nucleotides, or a modified nucleotide is combined with one or more unmodified nucleotides. The following Tables 1 and 2 present embodiments of oligonucleotides comprising modified nucleotides for example LNA which are indicated by (+) and phosphorothioate (PTO) indicated by (*). The oligonucleotides consisting of or comprising the sequences of Tables 1 and 2 may comprise any other modified nucleotide and any other combination of modified and unmodified nucleotides. Oligonucleotides of Table 1 hybridize with mRNA of human TDO2:

**Table 1: List of antisense oligonucleotides hybridizing with human TDO2 for example of SEQ ID No. 1; Neg1 is an antisense oligonucleotide representing a negative control which is not hybridizing with TDO2 of SEQ ID No. 1.**

| **SEQ ID No.** | **Name** | **Antisense Sequence 5'-3'** | **Antisense Sequence 5'-3' with PTO (*) and LNA (+)** |
|---|---|---|---|
| 3 | A07006H | TGTATGACAGCCGT | +T*+G*+T*A*T*G*A*C*A*G*C*+C*+G*+T |
| 4 | A07058H | ATCGTGGTGCTGAACAA | +A*+T*+C*G*T*G*G*T*G*C*T*G*A*A*+C*+A*+A |
| 5 | A07001H | GTATCCAGTGTCG | +G*+T*A*T*C*C*A*G*T*G*+T*+C*+G |
| 6 | A07002H | CTCGAACAGAATCC | +C*+T*+C*G*A*A*C*A*G*A*A*T*+C*+C |
| 7 | A07003H | TCTCGAACAGAATC | +T*+C*+T*C*G*A*A*C*A*G*A*+A*+T*+C |
| 8 | A07004H | TCGGAATTGCAAAC | +T*+C*+G*G*A*A*T*T*G*C*A*+A*+A*+C |
| 9 | A07005H | GAAGTTATCACGAT | +G*+A*+A*G*T*T*A*T*C*A*C*+G*+A*+T |
| 10 | A07007H | ACCGGTGCCAGCTT | +A*C*C*G*G*T*G*C*C*A*G*+C*+T*+T |
| 11 | A07008H | CATCTTCGGTATCC | +C*+A*+T*C*T*T*C*G*G*T*A*+T*+C*+C |
| 12 | A07009H | TCGTGGTGCTGAAC | +T*+C*G*T*G*G*T*G*C*T*G*A*A*+C |
| 13 | A07010H | TATCATTGACCTTCC | +T*+A*+T*C*A*T*T*G*A*C*C*T*+T*+C*+C |
| 14 | A07011H | ACCCACTCATGGTGA | +A*C*+C*C*A*C*T*C*A*T*G*G*T*+G*+A |
| 15 | A07012H | GATAAGACCTCCTTT | +G*+A*+T*A*A*G*A*C*C*T*C*C*+T*+T*+T |
| 16 | A07013H | CCAAATGCAGGTAGT | +C*+C*+A*A*A*T*G*C*A*G*G*T*+A*+G*+T |
| 17 | A07014H | GGATTTGCTTAAACC | +G*G*+A*T*T*T*G*C*T*T*A*A*+A*+C*+C |
| 18 | A07015H | CTCTCGAACAGAATC | +C*+T*+C*T*C*G*A*A*C*A*G*A*+A*+T*+C |
| 19 | A07016H | TCCGAGAAACAACCT | +T*+C*C*G*A*G*A*A*A*C*A*A*+C*+C*+T |
| 20 | A07017H | GATCACTGACACTCG | +G*+A*+T*C*A*C*T*G*A*C*A*C*+T*+C*+G |
| 21 | A07018H | TCGGAATTGCAAACT | +T*+C*+G*G*A*A*T*T*G*C*A*A*+A*+C*+T |
| 22 | A07019H | ACGATAATGTCTTCT | +A*+C*+G*A*T*A*A*T*G*T*C*T*+T*+C*+T |
| 23 | A07020H | ATGTGGCTCTAAACC | +A*+T*+G*T*G*G*C*T*C*T*A*A*+A*+C*+C |
| 24 | A07021H | TAGCCTGAATCCTTA | +T*+A*+G*C*C*T*G*A*A*T*C*C*+T*+T*+A |
| 25 | A07022H | GACAGTAGCACCTCT | +G*A*C*A*G*T*A*G*C*A*C*C*+T*C*+T |
| 26 | A07023H | GATGTTCATGACGTT | +G*A*+T*G*T*T*C*A*T*G*A*C*+G*+T*+T |
| 27 | A07024H | TCTGTATGACAGCCG | +T*+C*+T*G*T*A*T*G*A*C*A*G*+C*+C*+G |
| 28 | A07025H | AGTGAATCTATGTCC | +A*+G*+T*G*A*A*T*C*T*A*T*G*T*+C*+C |
| 29 | A07026H | CATGGTTATATCTCC | +C*+A*+T*G*G*T*T*A*T*A*T*C*+T*+C*+C |
| 30 | A07027H | ACCGGTGCCAGCTTT | +A*C*C*G*G*T*G*C*C*A*G*C*T*+T*+T |
| 31 | A07028H | GAACCACCGGTGCCA | +G*+A*+A*C*C*A*C*C*G*G*T*G*C*+C*+A |
| 32 | A07029H | TCGGTATCCAGTGTC | +T*+C*G*G*T*A*T*C*C*A*G*T*G*T*+C |
| 33 | A07030H | AGGAGCTATCACAGT | +A*+G*+G*A*G*C*T*A*T*C*A*C*+A*+G*+T |
| 34 | A07031H | TCAGAGCATCGTGGT | +T*+C*+A*G*A*G*C*A*T*C*G*T*+G*+G*+T |
| 35 | A07032H | CCCTATGTACAATGT | +C*+C*C*T*A*T*G*T*A*C*A*A*+T*+G*+T |
| 36 | A07033H | TCTCTCGAACAGAATC | +T*+C*+T*C*T*C*G*A*A*C*A*G*A*+A*+T*+C |
| 37 | A07034H | ATCCGAGAAACAACCT | +A*+T*+C*C*G*A*G*A*A*A*C*A*A*C*+C*+T |
| 38 | A07035H | GACACTCGGTGCATCC | +G*A*C*A*C*T*C*G*G*T*G*C*A*T*+C*+C |
| 39 | A07036H | ATCACTGACACTCGGT | +A*T*+C*A*C*T*G*A*C*A*C*T*C*+G*G*+T |
| 40 | A07037H | GTCGGAATTGCAAACT | +G*+T*+C*G*G*A*A*T*T*G*C*A*A*A*+C*+T |
| 41 | A07038H | GAGATGTTCATGACGT | +G*+A*+G*A*T*G*T*T*C*A*T*G*A*+C*+G*+T |
| 42 | A07039H | GTATGACAGCCGTCTT | +G*+T*+A*T*G*A*C*A*G*C*C*G*T*C*+T*+T |
| 43 | A07040H | CTCTGTATGACAGCCG | +C*+T*+C*T*G*T*A*T*G*A*C*A*G*+C*+C*+G |
| 44 | A07041H | ACCGGTGCCAGCTTTG | +A*+C*C*G*G*T*G*C*C*A*G*C*T*T*T*+G |
| 45 | A07042H | GATAGCCTGAGGAACC | +G*+A*T*A*G*C*C*T*G*A*G*G*A*+A*C*+C |
| 46 | A07043H | TTCGGTATCCAGTGTC | +T*T*+C*G*G*T*A*T*C*C*A*G*T*G*+T*+C |
| 47 | A07044H | TTCATCTTCGGTATCC | +T*+T*C*A*T*C*T*T*C*G*G*T*A*+T*+C*+C |
| 48 | A07045H | GCTATCATTGACCTTCC | +G*+C*+T*A*T*C*A*T*T*G*A*C*C*T*+T*+C*+C |
| 49 | A07046H | CTCTCGAACAGAATCCA | +C*+T*+C*T*C*G*A*A*C*A*G*A*A*T*+C*+C*+A |
| 50 | A07047H | TCCGAGAAACAACCTTA | +T*+C*+C*G*A*G*A*A*A*C*A*A*C*C*T*+T*+A |
| 51 | A07048H | ACTGACACTCGGTGCAT | +A*C*+T*G*A*C*A*C*T*C*G*G*T*G*+C*+A*+T |
| 52 | A07049H | GTCGGAATTGCAAACTC | +G*+T*C*G*G*A*A*T*T*G*C*A*A*A*C*+T*+C |
| 53 | A07050H | GTTATCACGATAATGTC | +G*T*+T*A*T*C*A*C*G*A*T*A*A*T*+G*+T*+C |
| 54 | A07051H | ATAAGGACAGTAGCACC | +A*+T*+A*A*G*G*A*C*A*G*T*A*G*C*+A*+C*+C |
| 55 | A07052H | GTTCATGACGTTTCTCA | +G*+T*+T*C*A*T*G*A*C*G*T*T*T*C*+T*+C*+A |
| 56 | A07053H | TGTATGACAGCCGTCTT | +T*+G*+T*A*T*G*A*C*A*G*C*C*G*T*+C*+T*+T |
| 57 | A07054H | TGTCCATAAGAGAAGTC | +T*+G*+T*C*C*A*T*A*A*G*A*G*A*A*+G*+T*+C |
| 58 | A07055H | ACCGGTGCCAGCTTTGC | +A*C*C*G*+G*T*G*C*C*A*G*C*T*T*T*G*+C |
| 59 | A07056H | ACCTATCACTCACAGTT | +A*+C*C*T*A*T*C*A*C*T*C*A*C*A*G*+T*+T |
| 60 | A07057H | TTCATCTTCGGTATCCA | +T*+T*+C*A*T*C*T*T*C*G*G*T*A*T*+C*+C*+A |
| 61 | A07059H | CCCTATGTACAATGTTA | +C*+C*+C*T*A*T*G*T*A*C*A*A*T*G*+T*+T*+A |
| 62 | Neg1 | | +C*+G*+T*T*T*A*G*G*C*T*A*T*G*T*A*+C*+T*+T |

The following Table 2 shows oligonucleotides hybridizing with mRNA of murine and/or rat TDO2:

**Table 2: List of antisense oligonucleotides hybridizing with murine and/or rat TDO2 for example of SEQ ID No. 2; Neg1 is an antisense oligonucleotide representing a negative control which is not hybridizing with TDO2 of SEQ ID No. 2.**

| **SEQ ID No.** | **Name** | **Antisense Sequence 5'-3'** | **Antisense Sequence 5'-3' with PTO (*) and LNA (+)** |
|---|---|---|---|
| 63 | A07002MR | TCGTCGTTCACCTTT | +T*+C*+G*T*C*G*T*T*C*A*C*C*+T*+T*+T |
| 64 | A07007MR | ATCACGATAGTGTTTC | +A*+T*+C*A*C*G*A*T*A*G*T*G*T*T*+T*+C |
| 65 | A07008MR | GTTATCACGATAGTGT | +G*+T*+T*A*T*C*A*C*G*A*T*A*G*+T*+G*+T |
| 66 | A07010MR | TCGTCGTTCACCTTTA | +T*+C*+G*T*C*G*T*T*C*A*C*C*T*+T*+T*+A |
| 67 | A07023MR | ATCACGATAGTGTTTCC | +A*+T*+C*A*C*G*A*T*A*G*T*G*T*T*T*+C*+C |
| 68 | A07001MR | GTAGTCATGACGCTT | +G*+T*+A*G*T*C*A*T*G*A*C*G*+C*+T*+T |
| 69 | A07003MR | CAGTCGTCGTTCACC | +C*+A*+G*T*C*G*T*C*G*T*T*C*+A*+C*+C |
| 70 | A07004MR | ACAGTCGTCGTTCAC | +A*+C*+A*G*T*C*G*T*C*G*T*T*+C*+A*+C |
| 71 | A07005MR | CCATAGATAAGTCCTC | +C*+C*+A*T*A*G*A*T*A*A*G*T*C*+C*+T*+C |
| 72 | A07006MR | CGAGAACTGCTGTACC | +C*+G*A*G*A*A*C*T*G*C*T*G*T*+A*+C*+C |
| 73 | A07009MR | CGTCGTTCACCTTTAC | +C*+G*+T*C*G*T*T*C*A*C*C*T*T*+T*+A*+C |
| 74 | A07011MR | AGTCGTCGTTCACCTT | +A*+G*+T*C*G*T*C*G*T*T*C*A*C*+C*+T*+T |
| 75 | A07012MR | CAGTCGTCGTTCACCT | +C*+A*+G*T*C*G*T*C*G*T*T*C*A*+C*+C*+T |
| 76 | A07013MR | GGTATGACAGTCGTCG | +G*+G*+T*A*T*G*A*C*A*G*T*C*G*+T*+C*+G |
| 77 | A07014MR | CACGGTATGACAGTCG | +C*+A*+C*G*G*T*A*T*G*A*C*A*G*+T*+C*+G |
| 78 | A07015MR | TGCACGGTATGACAGT | +T*+G*+C*A*C*G*G*T*A*T*G*A*C*+A*+G*+T |
| 79 | A07016MR | GGAGTGCACGGTATGA | +G*+G*+A*G*T*G*C*A*C*G*G*T*A*+T*+G*+A |
| 80 | A07017MR | GTACCTGTCGCTCACA | +G*+T*+A*C*C*T*G*T*C*G*C*T*C*+A*+C*+A |
| 81 | A07018MR | CTTGTACCTGTCGCTC | +C*+T*+T*G*T*A*C*C*T*G*T*C*G*+C*+T*+C |
| 82 | A07019MR | ACCTTGTACCTGTCGC | +A*+C*+C*T*T*G*T*A*C*C*T*G*T*+C*+G*+C |
| 83 | A07020MR | AATGATCGGATTCATC | +A*+A*+T*G*A*T*C*G*G*A*T*T*C*+A*+T*+C |
| 84 | A07021MR | GATTCATCGCTGCTGA | +G*+A*+T*T*C*A*T*C*G*C*T*G*C*T*+G*+A |
| 85 | A07022MR | CATAGATAAGTCCTCCT | +C*+A*+T*A*G*A*T*A*A*G*T*C*C*T*+C*+C*+T |
| 86 | A07024MR | TCGTCGTTCACCTTTAC | +T*+C*+G*T*C*G*T*T*C*A*C*C*T*T*+T*+A*+C |
| 87 | A07025MR | CAGTCGTCGTTCACCTT | +C*+A*+G*T*C*G*T*C*G*T*T*C*A*C*+C*+T*+T |
| 88 | A07026MR | ACAGTCGTCGTTCACCT | +A*+C*+A*G*T*C*G*T*C*G*T*T*C*A*+C*+C*+T |
| 89 | A07027MR | TGTGAATGATCGGATTC | +T*+G*+T*G*A*A*T*G*A*T*C*G*G*A*+T*+T*+C |
| 90 | Neg1 | | +C*+G*+T*T*T*A*G*G*C*T*A*T*G*T*A*+C*+T*+T |

The oligonucleotides of the present invention hybridize for example with mRNA of human or murine TDO of SEQ ID No. 1 and/or SEQ ID No. 2. Such oligonucleotides are called TDO, in particular TDO2 antisense oligonucleotides. In some embodiments, the oligonucleotides hybridize within position 1 to 1492 of TDO2 mRNA for example of SEQ ID No. 1.

In some embodiments, the oligonucleotide of the present invention inhibits at least about 50%, 55%, 60%, 65%, 70%, 75%, 80%, 85%, 90%, 92%, 94%, 95%, 96%, 97%, 98%, 99% or 100% of TDO such as the, e.g., human, murine TDO-2 expression. Thus, the oligonucleotides of the present invention are immunosuppression-reverting oligonucleotides which revert immunosuppression for example in a cell, tissue, organ, or a subject. The oligonucleotide of the present invention inhibits the expression of TDO such as TDO-2 at a nanomolar or micromolar concentration for example in a concentration of 0.1, 1, 2, 3, 4, 5, 6, 7, 8, 9, 10, 15, 20, 25, 30, 35, 40, 45, 50, 55, 60, 65, 70, 75, 80, 85, 90, 95, 100, 150, 200, 250, 300, 350, 400, 450, 500, 550, 600, 650, 700, 750, 800, 850, 900 or 950 nM, or 1, 10 or 100 µM.

In some embodiments, the oligonucleotide of the present invention is used in a concentration of 1, 3, 5, 9, 10, 15, 27, 30, 40, 50, 75, 82, 100, 250, 300, 500, or 740 nM, or 1, 2.2, 3, 5, 6.6 or 10 µM.

In some embodiments the present invention refers to a pharmaceutical composition comprising an oligonucleotide of the present invention and a pharmaceutically acceptable carrier, excipient and/or diluent. In some embodiments, the pharmaceutical composition further comprises a chemotherapeutic, another oligonucleotide, an antibody and/or a small molecule.

In some embodiments, the oligonucleotide or the pharmaceutical composition of the present invention is for use in a method of preventing and/or treating a disorder. In some embodiments, the use of the oligonucleotide or the pharmaceutical composition of the present invention in a method of preventing and/or treating a disorder is combined with radiotherapy. The radiotherapy may be further combined with a chemotherapy (e.g., platinum, gemcitabine). The disorder is for example characterized by an TDO imbalance, i.e., the TDO level is increased in comparison to the level in a normal, healthy cell, tissue, organ or subject for example resulting in altered, e.g., pathological health conditions. The TDO level is for example increased by an increased TDO such as TDO-2 expression and activity, respectively. The TDO level can be measured by any standard method such as immunohistochemistry, western blot, quantitative real time PCR or QuantiGene assay known to a person skilled in the art.

An oligonucleotide or a pharmaceutical composition of the present invention is administered locally or systemically for example orally, sublingually, nasally, subcutaneously, intravenously, intraperitoneally, intramuscularly, intratumoral, intrathecal, transdermal, and/or rectal. Alternatively or in combination *ex vivo* treated immune cells are administered. The oligonucleotide is administered alone or in combination with another immunosuppression-reverting oligonucleotide of the present invention and optionally in combination with another compound such as another oligonucleotide, an antibody, a small molecule and/or a chemotherapeutic (e.g., platinum, gemcitabine). In some embodiments, another oligonucleotide (i.e., not being part of the present invention), the antibody, and/or the small molecule are effective in preventing and/or treating an autoimmune disorder, an immune disorder, a psychiatric disorder (e.g., depression, schizophrenia, bipolar disorders such as bipolar depression, Alzheimer's disease) and/or cancer.

As tryptophan is the precursor of serotonin, a neurotransmitter which is involved in causing mild to severe depression leading for example to symptoms such as anxiety, apathy, fear, feelings of worthlessness, insomnia and fatigue, TDO inhibitors such as TDO2 antisense oligonucleotides are effective as antidepressants. In some embodiments TDO antisense oligonucleotides of the present invention are administered alone for preventing and/or treating depression or in combination with another antidepressant such as a serotonin reuptake inhibitor, e.g., Alaproclate (GEA-654), Cericlamine (JO-1017), Citalopram (Celexa), Dapoxetine (Priligy), Escitalopram (Lexapro, Cipralex), Femoxetine (Malexil), Fluoxetine (Prozac), Fluvoxamine (Luvox), Ifoxetine (CGP-15,210-G), Indalpine (Upstene), Omiloxetine, Panuramine (WY-26,002), Paroxetine (Paxil, Seroxat), Pirandamine (AY-23,713), RTI-353, Sertraline (Zoloft, Lustral), Zimelidine (Normud, Zelmid) or a combination thereof.

An oligonucleotide or a pharmaceutical composition of the present invention is used for example in a method of preventing and/or treating a solid tumor or a hematologic tumor. Examples of cancers preventable and/or treatable by use of the oligonucleotide or pharmaceutical composition of the present invention are breast cancer, colorectal carcinoma, lung cancer, malignant melanoma, mesothelioma, lymphoma, skin cancer, bone cancer, prostate cancer, hepatocarcinoma, brain cancer, cancer of the larynx, liver, gall bladder, pancreas, testicular, rectum, parathyroid, thyroid, adrenal, neural tissue, head and neck, colon, stomach, bronchi, kidneys, basal cell carcinoma, neuroblastoma, squamous cell carcinoma, metastatic skin carcinoma, osteo sarcoma, Ewing's sarcoma, reticulum cell sarcoma, liposarcoma, leukemia,myeloma, giant cell tumor, small-cell lung tumor, islet cell tumor, primary brain tumor, meningioma, acute and chronic lymphocytic and granulocytic tumors, acute and chronic myeloid leukemia, hairy-cell tumor, adenoma, hyperplasia, medullary carcinoma, intestinal ganglioneuromas, Wilm's tumor, seminoma, ovarian tumor, leiomyomater tumor, cervical dysplasia, retinoblastoma, soft tissue sarcoma, malignant carcinoid, topical skin lesion, rhabdomyosarcoma, Kaposi's sarcoma, osteogenic sarcoma, malignant hypercalcemia, renal cell tumor, polycythaemia vera, adenocarcinoma, anaplastic astrocytoma, glioblastoma multiforma, leukemia, or epidermoid carcinoma.

In some embodiments two or more oligonucleotides of the present invention are administered together, at the same time point for example in a pharmaceutical composition or separately, or on staggered intervals. In other embodiments, one or more oligonucleotides of the present invention are administered together with another compound such as another oligonucleotide (i.e., not being part of the present invention), an antibody, a small molecule and/or a chemotherapeutic, at the same time point for example in a pharmaceutical composition or separately, or on staggered intervals. In some embodiments of these combinations, the immunosuppression-reverting oligonucleotide inhibits the expression and activity, respectively, of an immune suppressive factor and the other oligonucleotide (i.e., not being part of the present invention), the antibody and/or small molecule inhibits (antagonist) or stimulates (agonist) an immune modulatory factor. In some embodiments a TDO2 antisense oligonucleotide of the present invention is combined with an IDO1 antisense oligonucleotide for example for preventing and/or treating tumor growth. The immune suppressive factor and/or the immune stimulatory factor is for example IDO (e.g., IDO), CTLA-4, PD-1, PD-L1, LAG-3, VISTA, A2AR, BTLA, CD39, CD73, STAT3, TIM-3, MICA, NKG2A, KIR, TIGIT, CD96, TGF-beta, Ox40, GITR, CD27, CD160, 2B4 or 4-1BB. The immune suppressive factor is a factor whose expression and/or activity is increased in a cell, tissue, organ or subject. The immune modulatory factor is a factor whose level is increased or decreased in a cell, tissue, organ or subject depending on the cell, tissue, organ or subject and its individual conditions.

An antibody in combination with the oligonucleotide or the pharmaceutical composition of the present invention is for example an anti-PD-1 antibody, an anti-PD-L1 antibody, or a bispecific antibody. A small molecule in combination with the oligonucleotide or the pharmaceutical composition of the present invention is for example (E)-6-fluoro-3-[2-(3-pyridyl)vinyl]-1H-indole (680C91).
A subject of the present invention is for example a mammalian, a bird or a fish.

### Examples

The following examples illustrate different embodiments of the present invention, but the invention is not limited to these examples.

### Example 1: Design of human TDO2 antisense oligonucleotides

For the design of antisense oligonucleotides with specificity for human (h) TDO2 the hTDO2 mRNA sequence with SEQ ID No. 1 (seq. ref. ID NM_005651.3; **Fig. 1**) was used. 14, 15, 16 and 17mers were designed according to in-house criteria, neg1 (described in WO2014154843 A1) was used as control antisense oligonucleotide in all experiments (Table 1). The distribution of the antisense oligonucleotide binding sites on the hTDO2 mRNA is shown in **Fig. 2****.**

### Example 2: Efficacy screen of hTDO2 antisense oligonucleotides in human cancer cell lines

In order to analyze the efficacy of hTDO2 antisense oligonucleotides of the present invention with regard to the knockdown of hTDO2 mRNA expression in cancer cell lines, MDA-MB-453 (human mammary carcinoma) and A-172 (human glioblastoma) cells were treated with a single dose (concentration: 5µM without addition of any transfection reagent; this process is called gymnotic delivery) of the respective antisense oligonucleotide as shown in **Fig. 3A** and **3B****.** hTDO2 and HPRT1 or GAPDH mRNA expression was analyzed three to four days later using the QuantiGene Singleplex assay (Affymetrix) and hTDO2 expression values were normalized to HPRT1 or GAPDH values. As shown in **Fig. 3A** (MDA-MB-453 cells) potent antisense oligonucleotides were preselected and tested for their activity in A-172 cells (**Fig. 3B**).

Values of the mean normalized mRNA expression of hTDO2 compared to non-treated cells are listed for MDA-MB-453 (Table 3) and A-172 cells (Table 4) in the following:

**Table 3: List of the mean normalized hTDO2 mRNA expression values in antisense oligonucleotide-treated MDA-MD-453 cells compared to non-treated cells.**

| **ASO** | **Relative hTDO2 mRNA expression (compared to non treated cells)** |
|---|---|
| A07009H | 0,29 |
| A07006H | 0,38 |
| A07032H | 0,43 |
| A07014H | 0,43 |
| A07058H | 0,46 |
| A07053H | 0,46 |
| A07018H | 0,48 |
| A07051H | 0,48 |
| A07040H | 0,49 |
| A07031H | 0,52 |
| A07039H | 0,54 |
| A07013H | 0,54 |
| A07020H | 0,56 |
| A07037H | 0,57 |
| A07026H | 0,58 |
| A07043H | 0,6 |
| A07017H | 0,61 |
| A07030H | 0,62 |
| A07049H | 0,64 |
| A07016H | 0,64 |
| A07024H | 0,66 |
| A07029H | 0,68 |
| A07041H | 0,68 |
| A07008H | 0,69 |
| A07050H | 0,71 |
| A07057H | 0,72 |
| A07019H | 0,72 |
| A07005H | 0,74 |
| A07004H | 0,74 |
| A07001H | 0,74 |
| A07028H | 0,75 |
| A07022H | 0,76 |
| A07036H | 0,77 |
| A07048H | 0,77 |
| A07045H | 0,77 |
| A07015H | 0,78 |
| A07044H | 0,78 |
| A07012H | 0,78 |
| A07021H | 0,79 |
| A07056H | 0,79 |
| A07059H | 0,8 |
| A07052H | 0,8 |
| A07042H | 0,82 |
| A07010H | 0,82 |
| A07002H | 0,83 |
| A07034H | 0,85 |
| A07007H | 0,87 |
| A07055H | 0,89 |
| A07011H | 0,93 |
| A07047H | 0,95 |
| A07035H | 0,98 |
| A07025H | 0,99 |
| A07003H | 1 |
| A07038H | 1,03 |
| No ASO | 1,04 |
| A07027H | 1,07 |
| A07033H | 1,09 |
| A07046H | 1,1 |
| A07054H | 1,2 |
| A07023H | 1,22 |
| neg1 | 1,27 |

**Table 4: List of the mean normalized hTDO2 mRNA expression values in antisense oligonucleotide-treated A-172 cells compared to non-treated cells.**

| **ASO** | **Relative hTDO2 mRNA expression (compared to non treated cells)** |
|---|---|
| A07058H | 0,11 |
| A07006H | 0,24 |
| A07053H | 0,25 |
| A07031H | 0,32 |
| A07039H | 0,35 |
| A07040H | 0,41 |
| A07051H | 0,41 |
| A07032H | 0,5 |
| A07018H | 0,5 |
| A07014H | 0,54 |
| neg1 | 0,6 |
| A07009H | 0,67 |
| A07020H | 0,68 |
| A07013H | 0,7 |

### Example 3: Correlation analysis of antisense oligonucleotide efficacy in MDA-MB-453 and A-172 cells

To further select the candidates with the highest activity in both tested cell lines MDA-MB-453 and A-172 a correlation analysis was performed (data derived from **Fig. 3A** and **3B**). As depicted in **Fig. 4**, 2 potent antisense oligonucleotides for determination of IC₅₀ in MDA-MB-453 cells, namely A07006H (SEQ ID No. 3) and A07058H (SEQ ID No. 4) (marked in black) were selected. Importantly, the control antisense oligonucleotide neg1 had no negative influence on the expression of hTDO2 MDA-MB-453 cells and a moderate influence in A-172 cells.

### Example 4: IC₅₀ determination of selected hTDO2 antisense oligonucleotides in MDA-MB-453 cells (mRNA level)

In order to determine the IC₅₀ of the hTDO2 antisense oligonucleotides A07006H (SEQ ID No. 3) and A07058H (SEQ ID No. 4), MDA-MB-453 cells were treated with titrated amounts of the respective antisense oligonucleotide (concentrations: 10µM, 3µM, 1µM, 300nM, 100nM, 30nM, 10nM, 3nM). hTDO2 mRNA expression was analyzed after 3 days (**Fig. 5**). Resulting IC₅₀ values were as follows:
- A07006H: 364nM
- A07058H: 627nM

**Table 5 in the following shows IC₅₀ values and target inhibition of the above mentioned antisense oligonucleotides at titrated concentrations in MDA-MB-453 cells:**

| | | **Inhibition (%)** | | | | | | | |
|---|---|---|---|---|---|---|---|---|---|
| **ASO** | **IC₅₀ (nM)** | **10µM** | **3µM** | **1µM** | **300nM** | **100nM** | **30nM** | **10nM** | **3nM** |
| A07006H | 364 | 71 | 53 | 58 | 45 | 26 | 28 | 11 | 14 |
| A07058H | 627 | 73 | 60 | 39 | 39 | 0 | 0 | 17 | 15 |

### Example 5: Downstream effect of hTDO2 knockdown on Kynurenine production in MDA-MB-453 cells

Kynurenines (L-kynurenine, kynurenic acid, 3-hydroxykynurenine) are the major immunosuppressive molecules that are generated during tryptophan degradation by hTDO2. The first kynurenine that is produced during tryptophan degradation is L-kynurenine which can be detected in cell culture supernatants by an enzyme linked immunosorbent assay (ELISA) (L-Kynurenine ELISA kit, ImmuSmol). MDA-MB-453 cells were treated for three days with the antisense oligonucleotides A07006H (SEQ ID No. 3) and A07058H (SEQ ID No. 4) at different concentrations (10µM, 3µM, 1µM, 300nM, 100nM and 30nM). Medium was renewed and supplemented with fresh antisense oligonucleotide at the respective concentration and L-Tryptophan.

Supernatants were harvested 24h after medium change and L-kynurenine concentrations were analyzed by ELISA. Strikingly, L-kynurenine production was strongly and dose-dependently suppressed when cells were treated with A07006H (SEQ ID No. 3) and A07058H (SEQ ID No. 4) (**Fig**. **6A****).** A clear correlation between knockdown efficacy of hTDO2 on the mRNA level (**Fig. 6B**) and Kynurenine level (**Fig. 6A**) in the supernatant could be observed. Treatment of MDA-MB-453 cells with antisense oligonucleotide had no influence on the cell viability (**Fig. 6C**).

**Table 6 in the following shows IC₅₀ values and % inhibition of L-Kynurenine production in MDA-MB-453 cells treated with the respective ASO at different concentrations:**

| | | **Inhibition (%)** | | | | | |
|---|---|---|---|---|---|---|---|
| **ASO** | **IC₅₀ (nM)** | **10µM** | **3µM** | **1µM** | **300nM** | **100nM** | **30nM** |
| A07006H | 1216 | 83 | 71 | 47 | 19 | 9 | 13 |
| A07058H | 920 | 72 | 57 | 39 | 25 | 8 | 0 |

### Example 6: Design of mouse/rat TDO2 antisense oligonucleotides

For the design of antisense oligonucleotides with specificity for murine (m) TDO2 the mTDO2 mRNA sequence with SEQ ID No. 2 (seq. ref. ID NM_019911; Fig. 7) was used. 15, 16 and 17mers were designed according to in-house criteria, neg1 (described in WO2014154843 A1) was used as control antisense oligonucleotide in all experiments (Table 1). The distribution of the antisense oligonucleotide binding sites on the mTDO2 mRNA is shown in **Fig. 8****.**

### Example 7: Efficacy screen of mTDO2 antisense oligonucleotides in a DasherGFP reporter system in HeLa cells

As there is no murine cancer cell line with TDO2 expression available, a reporter system based on a Dasher GFP reporter was designed that was fused to a fragment of the murine TDO2 gene. HeLa cells were transfected with this reporter construct and cells were treated with murine TDO2 antisense oligonucleotides at a concentration of 100nM. DasherGFP fluorescence intensity was measured after 24 hours (**Fig. 9**).

### Example 8: IC₅₀ determination of selected mTDO2 antisense oligonucleotides in a DasherGFP reporter system

In order to determine the IC₅₀ values of the mTDO2-specific ASOs A07002MR (SEQ ID No. 63), A07007MR (SEQ ID No. 64), A07008MR (SEQ ID No. 65), A07010MR (SEQ ID No. 66) and A07023MR (SEQ ID No. 67), HeLa cells were transduced with a DasherGFP/mTDO2 reporter construct and treated with different concentrations of the respective antisense oligonucleotide (100nM, 20nM, 4nM, 800pM, 160pM, 32pM) and the control antisense oligonucleotide neg1. DasherGFP fluorescence intensity was measured after 24 hours (**Fig**. **10**).

**Table 7 in the following shows IC₅₀ values and % inhibition of DasherGFP fluorescence intensity compared to untreated HeLa cells:**

| | | **Inhibition (%)** | | | | | |
|---|---|---|---|---|---|---|---|
| **ASO** | **IC₅₀ (nM)** | **100nM** | **20nM** | **4nM** | **800pM** | **160pM** | **32pM** |
| A07002MR | 2,5 | 98 | 96 | 69 | 3 | 0 | 2 |
| A07007MR | 0,16 | 100 | 99 | 89 | 58 | 33 | 0 |
| A07008MR | 0,83 | 100 | 99 | 85 | 47 | 31 | 7 |
| A07010MR | 2 | 98 | 95 | 53 | 2 | 0 | 0 |
| A07023MR | 0,5 | 99 | 100 | 95 | 60 | 23 | 1 |

## Claims

1. An immunosuppression-reverting oligonucleotide comprising 12 to 18 nucleotides, wherein at least one of the nucleotides is modified, and the oligonucleotide hybridizes with a nucleic acid sequence of tryptophan-2,3-dioxygenase (TDO2) of SEQ ID NO.1 (human) and/or of SEQ ID NO.2 (mouse), wherein the oligonucleotide inhibits at least 50 % of the TDO2 expression.

2. The oligonucleotide of claim 1, wherein the modified nucleotide is selected from the group consisting of a bridged nucleic acid such as LNA, cET, ENA, 2'Fluoro modified nucleotide, 2'O-Methyl modified nucleotide and a combination thereof.

3. The oligonucleotide of claim 1 or 2 hybridizing with TDO-2 of SEQ ID.NO.1 comprising a sequence selected from the group consisting of SEQ ID NO.3, SEQ ID NO.4, SEQ ID NO.5, SEQ ID NO.6, SEQ ID NO.7, SEQ ID NO.8, SEQ ID NO.9, SEQ ID NO.10, SEQ ID NO.11, SEQ ID NO.12, SEQ ID NO.13, SEQ ID NO.14, SEQ ID NO.15, SEQ ID NO.16, SEQ ID NO.17, SEQ ID NO.18, SEQ ID NO.19, SEQ ID NO.20, SEQ ID NO.21, SEQ ID NO.22, SEQ ID NO.23, SEQ ID NO.24, SEQ ID NO.25, SEQ ID NO.26, SEQ ID NO.27, SEQ ID NO.28, SEQ ID NO.29, SEQ ID NO.30, SEQ ID NO.31, SEQ ID NO.32, SEQ ID NO.33, SEQ ID NO.34, SEQ ID NO.35, SEQ ID NO.36, SEQ ID NO.37, SEQ ID NO.38, SEQ ID NO.39, SEQ ID NO.40, SEQ ID NO.41, SEQ ID NO.42, SEQ ID NO.43, SEQ ID NO.44, SEQ ID NO.45, SEQ ID NO.46, SEQ ID NO.47, SEQ ID NO.48, SEQ ID NO.49, SEQ ID NO.50, SEQ ID NO.51, SEQ ID NO.52, SEQ ID NO.53, SEQ ID NO.54, SEQ ID NO.55, SEQ ID NO.56, SEQ ID NO.57, SEQ ID NO.58, SEQ ID NO.59, SEQ ID NO.60, SEQ ID NO.61 and a combination thereof,
and/or
hybridizing with TDO-2 of SEQ ID.NO.2 comprising a sequence selected from the group consisting of SEQ ID NO.63, SEQ ID NO.64, SEQ ID NO.65, SEQ ID NO.66, SEQ ID NO.67, SEQ ID NO.68, SEQ ID NO.69, SEQ ID NO.70, SEQ ID NO.71, SEQ ID NO.72, SEQ ID NO.73, SEQ ID NO.74, SEQ ID NO.75, SEQ ID NO.76, SEQ ID NO.77, SEQ ID NO.78, SEQ ID NO.79, SEQ ID NO.80, SEQ ID NO.81, SEQ ID NO.82, SEQ ID NO.83, SEQ ID NO.84, SEQ ID NO.85, SEQ ID NO.86, SEQ ID NO.87, SEQ ID NO.88, SEQ ID NO.89 and a combination thereof.

4. The oligonucleotide of any one of claims 1 to 3, wherein the oligonucleotide is selected from the group consisting of +T*+G*+T*A*T*G*A*C*A*G*C*+C*+G*+T (A07006H), +A*+T*+C*G*T*G*G*T*G*C*T*G*A*A*+C*+A*+A (A07058H), +G*+T*A*T*C*C*A*G*T*G*+T*+C*+G (A07001H), +C*+T*+C*G*A*A*C*A*G*A*A*T*+C*+C (A07002H), +T*+C*+T*C*G*A*A*C*A*G*A*+A*+T*+C (A07003H), +T*+C*+G*G*A*A*T*T*G*C*A*+A*+A*+C (A07004H), +G*+A*+A*G*T*T*A*T*C*A*C*+G*+A*+T (A07005H), +A*C*C*G*G*T*G*C*C*A*G*+C*+T*+T (A07007H), +C*+A*+T*C*T*T*C*G*G*T*A*+T*+C*+C (A07008H), +T*+C*G*T*G*G*T*G*C*T*G*A*A*+C (A07009H), +T*+A*+T*C*A*T*T*G*A*C*C*T*+T*+C*+C (A07010H), +A*C*+C*C*A*C*T*C*A*T*G*G*T*+G*+A (A07011H), +G*+A*+T*A*A*G*A*C*C*T*C*C*+T*+T*+T (A07012H), +C*+C*+A*A*A*T*G*C*A*G*G*T*+A*+G*+T (A07013H), +G*G*+A*T*T*T*G*C*T*T*A*A*+A*+C*+C (A07014H), +C*+T*+C*T*C*G*A*A*C*A*G*A*+A*+T*+C (A07015H), +T*+C*C*G*A*G*A*A*A*C*A*A*+C*+C*+T (A07016H), +G*+A*+T*C*A*C*T*G*A*C*A*C*+T*+C*+G (A07017H), +T*+C*+G*G*A*A*T*T*G*C*A*A*+A*+C*+T (A07018H), +A*+C*+G*A*T*A*A*T*G*T*C*T*+T*+C*+T (A07019H), +A*+T*+G*T*G*G*C*T*C*T*A*A*+A*+C*+C (A07020H), +T*+A*+G*C*C*T*G*A*A*T*C*C*+T*+T*+A (A07021H), +G*A*C*A*G*T*A*G*C*A*C*C*+T*C*+T (A07022H), +G*A*+T*G*T*T*C*A*T*G*A*C*+G*+T*+T (A07023H), +T*+C*+T*G*T*A*T*G*A*C*A*G*+C*+C*+G (A07024H), +A*+G*+T*G*A*A*T*C*T*A*T*G*T*+C*+C (A07025H), +C*+A*+T*G*G*T*T*A*T*A*T*C*+T*+C*+C (A07026H), +A*C*C*G*G*T*G*C*C*A*G*C*T*+T*+T (A07027H), +G*+A*+A*C*C*A*C*C*G*G*T*G*C*+C*+A (A07028H), +T*+C*G*G*T*A*T*C*C*A*G*T*G*T*+C (A07029H), +A*+G*+G*A*G*C*T*A*T*C*A*C*+A*+G*+T (A07030H), +T*+C*+A*G*A*G*C*A*T*C*G*T*+G*+G*+T (A07031H), +C*+C*C*T*A*T*G*T*A*C*A*A*+T*+G*+T (A07032H), +T*+C*+T*C*T*C*G*A*A*C*A*G*A*+A*+T*+C (A07033H), +A*+T*+C*C*G*A*G*A*A*A*C*A*A*C*+C*+T (A07034H), +G*A*C*A*C*T*C*G*G*T*G*C*A*T*+C*+C (A07035H), +A*T*+C*A*C*T*G*A*C*A*C*T*C*+G*G*+T (A07036H), +G*+T*+C*G*G*A*A*T*T*G*C*A*A*A*+C*+T (A07037H), +G*+A*+G*A*T*G*T*T*C*A*T*G*A*+C*+G*+T (A07038H), +G*+T*+A*T*G*A*C*A*G*C*C*G*T*C*+T*+T (A07039H), +C*+T*+C*T*G*T*A*T*G*A*C*A*G*+C*+C*+G (A07040H), +A*+C*C*G*G*T*G*C*C*A*G*C*T*T*T*+G (A07041H), +G*+A*T*A*G*C*C*T*G*A*G*G*A*+A*C*+C (A07042H), +T*T*+C*G*G*T*A*T*C*C*A*G*T*G*+T*+C (A07043H), +T*+T*C*A*T*C*T*T*C*G*G*T*A*+T*+C*+C (A07044H), +G*+C*+T*A*T*C*A*T*T*G*A*C*C*T*+T*+C*+C (A07045H), +C*+T*+C*T*C*G*A*A*C*A*G*A*A*T*+C*+C*+A (A07046H), +T*+C*+C*G*A*G*A*A*A*C*A*A*C*C*T*+T*+A (A07047H), +A*C*+T*G*A*C*A*C*T*C*G*G*T*G*+C*+A*+T (A07048H), +G*+T*C*G*G*A*A*T*T*G*C*A*A*A*C*+T*+C (A07049H), +G*T*+T*A*T*C*A*C*G*A*T*A*A*T*+G*+T*+C (A07050H), +A*+T*+A*A*G*G*A*C*A*G*T*A*G*C*+A*+C*+C (A07051H), +G*+T*+T*C*A*T*G*A*C*G*T*T*T*C*+T*+C*+A (A07052H), +T*+G*+T*A*T*G*A*C*A*G*C*C*G*T*+C*+T*+T (A07053H), +T*+G*+T*C*C*A*T*A*A*G*A*G*A*A*+G*+T*+C (A07054H), +A*C*C*G*+G*T*G*C*C*A*G*C*T*T*T*G*+C (A07055H), +A*+C*C*T*A*T*C*A*C*T*C*A*C*A*G*+T*+T (A07056H), +T*+T*+C*A*T*C*T*T*C*G*G*T*A*T*+C*+C*+A (A07057H), +C*+C*+C*T*A*T*G*T*A*C*A*A*T*G*+T*+T*+A (A07059H) and a combination thereof,
and/or +T*+C*+G*T*C*G*T*T*C*A*C*C*+T*+T*+T (A07002MR), +A*+T*+C*A*C*G*A*T*A*G*T*G*T*T*+T*+C (A07007MR), +G*+T*+T*A*T*C*A*C*G*A*T*A*G*+T*+G*+T (A07008MR), +T*+C*+G*T*C*G*T*T*C*A*C*C*T*+T*+T*+A (A07010MR), +A*+T*+C*A*C*G*A*T*A*G*T*G*T*T*T*+C*+C (A07023MR), +G*+T*+A*G*T*C*A*T*G*A*C*G*+C*+T*+T (A07001MR), +C*+A*+G*T*C*G*T*C*G*T*T*C*+A*+C*+C (A07003MR), +A*+C*+A*G*T*C*G*T*C*G*T*T*+C*+A*+C (A07004MR), +C*+C*+A*T*A*G*A*T*A*A*G*T*C*+C*+T*+C (A07005MR), +C*+G*A*G*A*A*C*T*G*C*T*G*T*+A*+C*+C (A07006MR), +C*+G*+T*C*G*T*T*C*A*C*C*T*T*+T*+A*+C (A07009MR), +A*+G*+T*C*G*T*C*G*T*T*C*A*C*+C*+T*+T (A07011MR), +C*+A*+G*T*C*G*T*C*G*T*T*C*A*+C*+C*+T (A07012MR), +G*+G*+T*A*T*G*A*C*A*G*T*C*G*+T*+C*+G (A07013MR), +C*+A*+C*G*G*T*A*T*G*A*C*A*G*+T*+C*+G (A07014MR), +T*+G*+C*A*C*G*G*T*A*T*G*A*C*+A*+G*+T (A07015MR), +G*+G*+A*G*T*G*C*A*C*G*G*T*A*+T*+G*+A (A07016MR), +G*+T*+A*C*C*T*G*T*C*G*C*T*C*+A*+C*+A (A07017MR), +C*+T*+T*G*T*A*C*C*T*G*T*C*G*+C*+T*+C (A07018MR), +A*+C*+C*T*T*G*T*A*C*C*T*G*T*+C*+G*+C (A07019MR), +A*+A*+T*G*A*T*C*G*G*A*T*T*C*+A*+T*+C (A07020MR), +G*+A*+T*T*C*A*T*C*G*C*T*G*C*T*+G*+A (A07021MR), +C*+A*+T*A*G*A*T*A*A*G*T*C*C*T*+C*+C*+T (A07022MR), +T*+C*+G*T*C*G*T*T*C*A*C*C*T*T*+T*+A*+C (A07024MR), +C*+A*+G*T*C*G*T*C*G*T*T*C*A*C*+C*+T*+T (A07025MR), +A*+C*+A*G*T*C*G*T*C*G*T*T*C*A*+C*+C*+T (A07026MR), +T*+G*+T*G*A*A*T*G*A*T*C*G*G*A*+T*+T*+C (A07027MR) and a combination thereof, wherein + indicates an LNA nucleotide and * indicates a phosphorothioate (PTO) linkage between the nucleotides.

5. The oligonucleotide of any one of claims 1 to 4, wherein the oligonucleotide inhibits the expression of TDO-2 at a nanomolar concentration.

6. The oligonucleotide of any one of claims 1 to 5, wherein the concentration of the oligonucleotide is 1 nM, 3 nM, 5 nM, 10 nM, 30 nM, 100 nM, 300 nM, 1 µM, 3 µM, 5 µM, 10 µM or 30 µM.

7. A pharmaceutical composition comprising an immunosuppression-reverting oligonucleotide of any one of claims 1 to 6 and a pharmaceutically acceptable carrier, excipient, diluent or a combination thereof.

8. The pharmaceutical composition of claim 7, further comprising a chemotherapeutic for example selected from the group consisting of platinum, gemcitabine, another oligonucleotide, an antibody and/or a small molecule being effective in tumor treatment.

9. The pharmaceutical composition of claim 7, wherein the immunosuppression-reverting oligonucleotide inhibits the expression of an immune suppressive factor, and the antibody and/or the small molecule inhibits or stimulates an immune modulatory factor.

10. The pharmaceutical composition of claim 9, wherein the immune suppressive factor and/or the immune modulatory factor is selected from the group consisting of CTLA-4, IDO, PD-1, PD-L1, LAG-3, VISTA, A2AR, BTLA, CD39, CD73, STAT3, TIM-3, MICA, NKG2A, KIR, TIGIT, CD96, TGF-beta, Ox40, GITR, CD27, CD160, 2B4, 4-1BB and a combination thereof.

11. The immunosuppression-reverting oligonucleotide of any one of claims 1 to 6 or the pharmaceutical composition of any one of claims 7 to 10 for use in a method of preventing and/or treating a disorder, where TDO imbalance is involved.

12. The immunosuppression-reverting oligonucleotide or the pharmaceutical composition for use according to claim 11, wherein the disorder is an autoimmune disorder, an immune disorder, a psychiatric disorder, infectious liver disease and/or cancer.

13. The immunosuppression-reverting oligonucleotide or the pharmaceutical composition for use according to claim 12, wherein the cancer is bladder carcinoma, breast cancer, colorectal carcinoma, lung cancer, malignant melanoma, mesothelioma, lymphoma, skin cancer, bone cancer, prostate cancer, hepatocarcinoma, brain cancer, cancer of the larynx, liver, gall bladder, pancreas, testicular, rectum, parathyroid, thyroid, adrenal, neural tissue, head and neck, colon, stomach, bronchi, kidneys, basal cell carcinoma, neuroblastoma, squamous cell carcinoma, metastatic skin carcinoma, osteo sarcoma, Ewing's sarcoma, reticulum cell sarcoma, liposarcoma, leukemia, myeloma, giant cell tumor, small-cell lung tumor, islet cell tumor, primary brain tumor, meningioma, acute and chronic lymphocytic and granulocytic tumors, acute and chronic myeloid leukemia, hairy-cell tumor, adenoma, hyperplasia, medullary carcinoma, intestinal ganglioneuromas, Wilm's tumor, seminoma, ovarian tumor, leiomyomater tumor, cervical dysplasia, retinoblastoma, soft tissue sarcoma, malignant carcinoid, topical skin lesion, rhabdomyosarcoma, Kaposi's sarcoma, osteogenic sarcoma, malignant hypercalcemia, renal cell tumor, polycythaemia vera, adenocarcinoma, anaplastic astrocytoma, glioblastoma multiforma, leukemia, or epidermoid carcinoma.

14. The immunosuppression-reverting oligonucleotide or the pharmaceutical composition for use according to any one of claims 11 to 13, wherein the oligonucleotide or the composition is administered locally or systemically.
